# NEW EUROPEAN PATENT SPECIFICATION

(11) **EP 0 107 209 B2**
(45) Date of publication and mention of the opposition decision: **11.12.1996**
(45) Mention of the grant of the patent: 24.09.1986
(21) Application number: 83110713.1
(22) Date of filing: 26.10.1983
(51) Int. Cl.: A61K 31/70

(54) **Sucralfate preparation**
Sucralfat-Präparat
Préparation contenant du sucralfate

(30) Priority: 27.10.1982 JP 187614/82
(43) Date of publication of application: 02.05.1984
(73) Proprietor: Chugai Seiyaku Kabushiki Kaisha, Tokyo (JP)
(72) Inventor: Ishihara, Kouji, Soka-shi Saitama-ken (JP); Igusa, Kazuo, Hachioji-shi Tokyo (JP); Ogasawara, Toshichika, Nakano-ku Tokyo (JP)
(74) Representative: VOSSIUS & PARTNER

(56) References cited:
- ARZNEIMITTEL-FORSCHUNG, vol. 29(II), no. 11, November 1979, pages 1668-1676, Aulendorf, DE. R. NAGASHIMA et al.: "Sucralfate, a basic aluminum salt of sucrose sulfate"
- ARZNEIMITTEL-FORSCHUNG, vol. 32(I), no. 5, May 1982, pages 512-518, Aulendorf, DE. K. STEINER et al.: "Sucralfate: Pharmacokinetics, metabolism and selective binding to experimental gastric and duodenal ulcers in animals"
- CHEMICAL ABSTRACTS, vol. 97, no. 15, 11th October 1982, page 17, no. 120076d, Columbus, Ohio, USA Y. HINOHARA et al.: "The affinity of Sucralfate to gatric mucosa in the model of experimental gastritis induced by ethanol and aspirin"
- CHEMICAL ABSTRACTS, vol. 97, no. 15, 11th October 1982, page 51, no. 120364w, Columbus, Ohio, USA Y. HINOHARA et al.: "Effects of Sucralfate on the permeability of gastric mucosa to iodine-125-labeled human serum albumin in experimental gastritis induced by ethanol"
- CHEMICAL ABSTRACTS, vol. 97, no. 15, 11th October 1982, page 51, no. 120365x, Columbus, Ohio, USA N. KOBATAKE et al.: "Metabolism of glycoproteins in gastric mucosa and effects of Sucralfate on ethanol-induced gastritis in rats"

## Description

The present invention relates to a sucralfate preparation containing an amino acid free from benzene rings and sulfur atoms in its chemical structure, except glycine.

An aluminium salt of sucrose sulfate ester (conventionally known as sucralfate) is an excellent ulcer preventing agent and because of its minimal side effects, is extensively used as an anti-ulcer agent.

Sucralfate is characterized by anti-pepsin and antacid effects in that it binds to pepsin and acid in gastric juice, which are two factors that attack the ulcer-affected area of digestive tracts, and thereby inhibits directly their activities. Sucralfate is also characterized by its ability to protect the mucosa in that it forms a sucralfate coat on the mucosa of the digestive tract and protects it from the attacking factors. These effects combine to provide sucralfate with high anti-ulcer activities. It has also been found that sucralfate is effective in revascularization and accelerating the growth of regenerated mucosa. and hence is considered to have a great potential for use as an agent to promote the healing of ulcers. Sucralfate has an ability to selectively bind to the ulcer-affected mucosa of the stomach and duodenum rather than the normal mucosa of the digestive tract and form a protective coat against the invasion of the attacking factors, and this ability is the most characteristic feature of sucralfate which is not found in other anti-ulcer agents. Sucralfate forms a sticky paste in an acidic solution at a pH below 4.0. When administered orally, sucralfate reacts with gastric juice in the stomach to form a sticky paste which binds to the ulcer-affected part of the stomach or duodenum to exhibit a sustained effect to protect the mucosa in that part. This effect of sucralfate can be confirmed in vitro by the following procedure:

According to the Japanese Pharmacopoeia (10th ed.), 1,000 ml of artificial gastric juice (pH: ca. 1.2) is prepared from a mixture of sodium chloride (2.0 g), dilute hydrochloric acid (24 ml) and water. Sucralfate (1 g) is suspended in 10 ml of the gastric juice in a test tube, and immediately thereafter, sucralfate forms a sticky paste which adheres to the inner surface of the test tube as if sucralfate administered orally contacted acid in the stomach and formed a viscous paste.

In the course of our studies on sucralfate in vitro, we have unexpectedly found that an aqueous suspension of sucralfate or a solid preparation thereof placed under hot and humid conditions loses partly, or entirely, the most characteristic feature of sucralfate (ability to form a sticky paste in the artificial gastric juice) although its other properties remain substantially unchanged. In particular, this phenomenon occurs in aqueous suspensions in 15 to 20 days after its preparation, and sucralfate particles remain suspended even if the suspension is mixed with the artificial gastric juice. We conducted animal experiments with rats in which ulcers were artificially induced, and confirmed that sucralfate preparations with reduced ability to form a viscous paste were not highly effective in protecting the ulcer-affected mucosa from attacking factors.

Therefore, we have made various efforts to produce a sucralfate preparation that keeps its ability to form a viscous paste in gastric juice over an extended period. As a result, we have found that this object can be achieved by adding specific amino acids to sucralfate. We continued our studies and have also found that the amino acids which are the most effective for this purpose are neutral amino acids free from benzene rings in their chemical structures, as well as acidic and basic amino acids free from benzene rings, and salts thereof. However, amino acids containing sulfur atoms in their chemical structures are not desirable since their mixtures with sucralfate develop a colour in the course of time.

The present invention has been accomplished on the basis of these findings, and relates to a sucralfate preparation containing an amino acid free from benzene rings or sulfur atoms in its chemical structure, except glycine. Suitable amino acids that may be used in the present invention include neutral amino acids such as alanine, valine, serine, proline, glutamine, asparagine, aminobutyric acid and ε-aminocaproic acid; acidic amino acids such as glutamic acid and aspartic acid and salts thereof; basic amino acids such as lysine, arginine and histidine and salts thereof. Also effective are compounds which contain these amino acid structures in their structure, such as pantothenic acid having the β-alanine moiety and salts thereof, and N-glycylglycine having the glycine moiety.

According to the present invention, sucralfate may be mixed with an amino acid in any proportions so long as the desired effect of the amino acid is attained by mixing. Generally, the amount of amino acid is about 0.5 to 50 wt% of sucralfate, and about 1 to 10 wt% is preferred. In order to form an aqueous suspension of sucralfate, a suitable amino acid may be dissolved in a dispersion medium together with sucralfate. If a solid preparation is desired, a uniform mixture of sucralfate and the amino acid is first prepared by dissolving the amino acid in water or a common binder, then sucralfate is mixed with the solution, and the mixture is finally formulated by a conventional technique to make a desired preparation in the form of tablets, capsules, granules or subtilized granules. The sucralfate preparations in either form proved to be highly stable for an extended period without losing the ability to form a sticky paste in artificial gastric juice. In addition, there was no decrease in the ability of sucralfate to protect the mucosa of the digestive tract in animals or humans.

The advantage of the present invention are hereunder described in greater detail by reference to the following non-limiting Examples.

### Example 1

To a mixture of sucralfate (100 g), glycerin (200 g), 70% D-sorbitol (200 g) and an antiseptic (1.2 g), distilled water was added to make 1000 ml. The mixture was well stirred with an agitating/homogenizing mixer to form a suspension. The suspension was divided into two equal portions (500 ml each). To one portion, β-alanine (2.5 g) was added and the mixture was well stirred. The two portions of the suspension were left to stand at room temperature for 30 days until they separated into liquid and solid phases. Each portion was stirred to restore a uniform suspension. Two milliliters off each suspension were placed in a test tube, and artificial gastric juice (20 ml) was added to each suspension, and the mixture was stirred. In the suspension containing β-alanine and sucralfate formed a sticky paste that adhered to the inner surface of the test tube and the supernatant remained almost clear. However, there was no change in the β-alanine free suspension and no deposit was formed on the inner surface of the test tube.

### Example 2

To a solution of L-proline (300 g) in pure water (11,000 ml), sucralfate (5000 g) was added, and the resulting dispersion was dried with a spray dryer to form a granular powder. As a control, a granular powder free from L-proline was prepared in the same manner. The two samples of granular powder were left to stand for 30 days at 40°C and 85% relative humidity. Thereafter, a 1 g portion of each sample was placed in a test tube, mixed with 10 ml of artificial gastric juice, and the mixture was stirred. With the sample containing L-proline, a sticky paste formed and adhered to the inner surface of the test tube. However, the granules of the control sample remained suspended in the gastric juice.

### Example 3

Sucralfate preparations containing amino acids indicated in the following table (for their amounts, also see the table) were formulated as in Example 1 and subjected to an accelerated storage test. In each sample, sucralfate exhibited sustained ability to form a sticky paste under acidic conditions.

**TABLE**

| Amino acids | Effective amount (wt% relative to sucralfate) |
|---|---|
| L-alanine | 0.5< |
| γ-aminobutyric acid (n) | |
| ε-aminocaproic acid | 1< |
| DL-alanine | |
| L-arginine | 3< |
| L-arginine hydrochloride | |
| L-lysine hydrochloride | |
| L-aspartic acid | |
| sodium L-aspartate | |
| L-glutamic acid | |
| L-histidine hydrochloride | |
| L-glutamine, L-asparagine, | 5< |
| L-serine, DL-valine, | |
| L-proline | |

## Claims

1. A sucralfate preparation containing an amino acid having no benzene rings or sulfur atoms in its chemical structure, except glycine.

2. A sucralfate preparation according to Claim 1 which contains 0.5 to 50% by weight of said amino acid based on the amount of sucralfate.

3. A sucralfate preparation according to Claim 2 which contains 1 to 10% by weight of said amino acid based on the amount of sucralfate.

## Patentansprüche

1. Sucralfat-Präparat enthaltend eine Aminosäure, die keine Benzolringe oder Schwefelatome in ihrer chemischen Strukturformel aufweist, ausgenommen Glycin.

2. Sucralfat-Präparat nach Anspruch 1, das 0,5 bis 50 Gewichtsprozent der Aminosäure bezogen auf die Menge des Sucralfats enthält.

3. Sucralfat-Präparat nach Anspruch 2, das 1 bis 10 Gewichtsprozent der Aminosäure bezogen auf die Menge des Sucralfats enthält.

## Revendications

1. Préparation à base de sucralfate contenant un aminoacide dont la structure chimique ne comporte pas de cycles benzéniques ou d'atomes de soufre, à l'exception de la glycine.

2. Préparation selon la revendication 1, contenant 0,5 à 50 % en poids dudit aminoacide par rapport à la quantité de sucralfate.

3. Préparation selon la revendication 2, contenant 1 à 10 % en poids dudit aminoacide par rapport à la quantité de sucralfate.
